Europäisches Patentamt

European Patent Office

Office européen des brevets

(11) Publication number: **0 355 882**
**A1**

## EUROPEAN PATENT APPLICATION

(21) Application number: **89201896.1**

(22) Date of filing: **19.07.89**

(51) Int. Cl.4: **C07K 3/20 , B01D 15/08 , C07K 15/14**

(30) Priority: **11.08.88 NL 8802003**

(43) Date of publication of application:
**28.02.90 Bulletin 90/09**

(84) Designated Contracting States:
**BE DE ES FR GB LU NL**

(71) Applicant: **NEDERLANDSE ORGANISATIE VOOR TOEGEPAST-NATUURWETENSCHAPPELIJK ONDERZOEK TNO**
**Juliana van Stolberglaan 148**
**NL-2595 CL The Hague(NL)**

(72) Inventor: **Doddema, Harmannus Johannes**
**Nieuwe Kade 17**
**NL-3511 RV Utrecht(NL)**

(54) Method for the preparation of fibronectine from blood plasma, with the help of affinity chromatography and the column material to be used with it.

(57) The invention deals with a method, for the preparation of fibronectine from blood plasma, with which gelatine is used as a ligand, attached to a carrier material. For this, blood plasma is led over an affinity chromatography column, made up of active carbon particles, to which gelatine is attached as a ligand, for matrix material; now fibronectine is attached to gelatine, and then the fibronectine is separated from the column in a way fit for affinity chromatography.

The invention also deals with a column material containing active carbon, to which gelatine is attached as a ligand, as matrix material, and also a method for the preparation of such material.

EP 0 355 882 A1

## Methods for the preparation of fibronectine from blood plasma, with the help of affinity chromatography and the column material to be used with it.

The invention deals with a method for the preparation of fibronectine from blood plasma with which gelatine is used as a ligand, combined with a carrier material.

Affinity chromatography is a form of adsorption chromatography in which a combination, to be extracted from a liquid solution, is adsorbed specifically and reversibly by a complementarily combining material (ligand), the latter being immobilized on an insoluble carrier (matrix).

Among well-known matrix materials are polysaccharides, particularly products derived from agarose (Note: "Affinity Chromatography" by Pharmacia Fine Chemicals). Axen, Porath and Ernback found that molecules containing primary amino groups as a ligand can be combined with polysaccharide matrices which are activated by a cyane halide (CNBr) (214 Nature 1302-1314 (1967)).

Furthermore, it is known that gelatine is a ligand fit for combining with crosslinked agarose pearls, for the separation of fibronectine from blood plasma (Bio-Rad Bulletin, 1084 june 1981). Fibronectine has become of importance with the cultivation of animal cells on micro-carriers for the production of medicine. For that reason, it is important that cheap and easily practicable methods become available for the production of fibronectine.

Affinity chromatography, with gelatine and agarose for column material, is a very useful technique for extracting fibronectine from blood plasma. A drawback however is that it is an expensive method as a result of the high cost price of the matrix material.

The invention aims at providing a cheap method for the preparation of fibronectine from blood plasma with the help of affinity chromatography, with which gelatine is used as ligand material, by applying a cheap matrix material.

The invention also deals with the column material to be applied with this method.

Surprisingly, it was found that active carbon, a product that, chemically, shows no affinity with the mentioned agarose products at all, makes a matrix material on which gelatine as a ligand can be immobilized while maintaining its specific and reversible adsorption abilities for fibronectine. Active carbon is a relatively cheap product which makes it an excellent matrix material for gelatine as a ligand, for the preparation of fibronectine from blood plasma.

The invention consists of a method for the preparation of fibronectine from blood plasma with the help of affinity chromatography, with which the ligand of the column material consists of gelatine, with the characteristic that blood plasma is led over an affinity chromatography column, made up of active carbon particles for matrix material, to which gelatine is attached as a ligand. The fibronectine is attached to the gelatine and the fibronectine, after which the fibronectine is separated from the column in a way fit for affinity chromatography.

The invention of this method can be explained as follows.

For blood plasma, the plasma of cattle, cows for instance, can be considered.

Furthermore, it can be observed that for the preparation of fibronectine from blood plasma as well as the preparation of the column material, use can be made of methods which, by themselves, are known from the technique of affinity chromatography. For these, one is directed to the literature mentioned above.

It is important to equilibrate the chromatography column in advance with a fit buffer solution to keep the plasma albumen, including the fibronectine, from denaturizing. This can be done easiest by rinsing the column with a watery, buffered, weakly alcalic solution, preferably a watery solution of ammoniumbicarbonate with a pH of 7-9, specifically a pH of 8.5, or with a phosphate buffer with 0.1 M NaCl-pH 7.5.

After the plasma is led over the column and the fibronectine present is attached to the gelatine, the column is rinsed with a watery solution in order to drain away albumen also left in the column. This is done preferably with the same solution the column was equilibrated with. This rinsing is done with so much solution until no more albumen come from the column. Then, the fibronectine is eluated from the column by rinsing it with a watery solution with a pH and the presence of soluded combinations, breaking the connection between the gelatine and the fibronectine. For this, a buffered watery solution is considered, with a pH of about the isoelectric point of gelatine, that is about 5.5. In general, good results are seen with a pH between 4 and 6. A watery, vinegar-acid/acetate solution is considered. Preferably, elements are added to the solution that help break the connection between the gelatine and the fibronectine. As such natriumbomide, glucose, ureum or glycine are mentioned. For this elution, as well as the other steps of the method according to the invention, one is referred to techniques, common to affinity chromatography. Note the book "Affinity Chromatography" and the "Bio-Rad Bulletin" mentioned above.

The further purification of the fibronectine solution and its isolation can be done using common techniques like gel filtration, ultra-filtration and the use of ion exchangers.

The invention also deals with a new column material for affinity chromatography.

The new column material is made up of active carbon particles to which the gelatine is attached adsorbtively. Active carbon is almost-pure carbon. It is available on the market. Usually, it has gone through one or more purification processes; for instance the raw carbon has been treated with oxygen and then gone through an acid extraction for the separation of elements like sulphur and heavy metals. Also, oxydation enlarges the number of adsorptive groups (capacity for attachment).

For the right active carbon for applying the invention, preferably active carbon type 500B by "NORIT" is used, because this type has a high capacity for attaching albumen.

The active carbon particles are porous and the pores are filled with gasses like air. Enclosed gas limits the adsorption capacity of gelatine and this the attachment to the matrix material. For this reason. it is recommended to de-gasify the active carbon before applying the method of this invention.

The invention further deals with a method for the preparation of the new column material.

This method is characterized thus, that a watery gelatine solution is mixed with a watery suspension of active carbon particles until the gelatine is adsorbed by the active carbon particles, after which superfluous gelatine is disposed of by washing it out.

According to the preferred way of the invention, for attaining the best attachment of gelatine to carbon, gelatine is soluded in a watery, buffered solution at 70 °C and at an ion strength of the solution between 0.01 and 0.1 M and a pH between 3 and 8, preferably about the iso-electric point of the gelatine (5.5), after which the watery gelatine solution is mixed with suspension of de-gasified particles of active carbon, this suspension being brought to the same ion strength and pH number as the gelatine solution, after which gelatine is allowed to attach itself to and in the matrix of active carbon particles at a temperature between 20 °C and 90 °C, preferably 70 °C, after which the superfluous gelatine is disposed of by washing it out.

The size of the particles of the new matrix materials does not lie between narrow boundaries. The size is determined by demands made of the speed of passage of a watery solution through a column of a watery suspension of the particles, and of the speed of diffusion of the reagentia in the matrix of the particles. Particles too small are disadvantageous to the speed of passage, particles too big are disadvantageous to the speed of diffusion. Preferably, the particle size is between 0.01 and 1 mm, specifically between 0.1 and 0.55 mm. For the new column material of the invention, preferably pure gelatine is used with less than 1% impurities.

The de-gasification of the carbon particles can be done easily by heating a watery suspension of the active carbon particles for a while, for instance between 50 °C and 100 °C, preferably 100 °C.

Also, it has been shown to be advantageous to conduct the de-gasification of the carbon particles under buffered circumstances, where the concentration of ions is kept low and the pH is kept constant. The pH preferably is about the iso-electric point of gelatine and the ion strength between 0.1 and 1 Mol. Those circumstances correspond to those where a good attachment between the gelatine and the active carbon particles is achieved.

The attachment of the gelatine to and in the active carbon particles takes a while. The time it takes depends upon the sort of gela tine used and the nature and size of the carbon particles. In general, it takes a period of 20 to 4 hours.

The disposal of non-attached gelatine can be done easily by filtering the suspension of the active carbon particles and the gelatine attached to it, and washing the residue in the filter with a buffered, watery solution of the same pH and ion strength as the solution for attaching the gelatine to the matrix mentioned before.

The new column material, according to the invention, can be marketed in the form of a watery suspension of gelatine adsorbed in active carbon particles, preferably with a low ion concentration and buffered at a pH about the iso-electric point of gelatine, but also in dried form. The dried column material, according to the invention, contains 10-50% gelatine, attached adsorbtively to carbon, 50-90% carbon particles and 0-10% iogenous ingredients.

Practical example:

10 g of so-called Sigma III Calfskin gelatine, obtained from Sigma Chemical Co., St. Louis - USA, catalogue no. G 0510 was soluded in 100 ml destilled water, to which 5 mMol (0.410 g) of natrium acetate was added (pH 5.5). The solution was put away at 75 °C;

10 g of active carbon particles, type 500B, obtained from NORIT (bars), average particle size 0.1 by 0.5 cm, was suspended in 100 ml of destilled water, to which 5 mMol (0.410 g) natrium acetate was added (pH 5.5). The suspension was boiled for 5 min. to dispose of the air in the pores. Then the suspension was cooled to room temperature and again put at pH 5.5 by titration with lye or acid (NaOH or HCl), until the pH had become stable in time, after which the suspension was heated again to 75 °C.

Then the warm gelatine solution was mixed with a warm suspension and the mixing was continued while stirring, for 24 hours at 75 °C.

After that, the column material was rinsed three times with 100 ml of destilled water, in which 5 mMol (0,410 g) natrium acetate was soluded, at a temperature of 75 °C on a Büchner funnel to dispose of non-adsorbed gelatine. At last, it was rinsed once again with 100 ml of the same watery natrium acetate solution at room temperature.

The column material, made thus and dried, in the amount of 5 g, was put in a glass column which has been treated with silicones on the inside, with a diameter of 3 cm and 6 cm high. The column, filled like this, was rinsed with a solution of 5 mMol (0.395 g) of ammoniumbicarbonate (pH 8.5), to which 0.25 mMol (1.410 g) of EDTA was added. Now the column was equilibrated and ready for the isolation of fibronectine from cow blood plasma.

Through the equilibrated column 64 ml of fresh cattle plasma is pumped, or enough to saturate the column with fibronectine. Then the column is rinsed with 66 ml 0.05 phosphate buffer, 0.9 NaCl pH 5 mMol EDTA pH 7.2, and 50 mM vinegar acid/acetate buffer 1 M NaCl pH 5, until no more albumen come from the column.

Fibronectine is now eluted from the column by rinsing with 50 ml 50 mMol vinegar acid/acetate buffer 1 M NaBr pH 5.0. The fibronectine is rinsed from the column with this buffer. (>90% pure, 50-90% yield). The fibronectine can be de-salted over a Sephadex G25 molecular screen, or any other way (ultra filtration, etc.) and can then be further purified (>97%) over an anion exchanger.

## Claims

1. Method for the preparation of fibronectine from blood plasma with the help of affinity chromatography, with which column material is used containing gelatine as a ligand, characterized in that blood plasma is led over an affinity chromatography column made up of active carbon particles, to which gelatine is attached as a ligand, for matrix material; now fibronectine is attached to the gelatine, and then the fibronectine is separated from the column in a way fit for affinity chromatography.

2. Column material for affinity chromatography containing gelatine as a ligand, characterized in that the column material contains active carbon parts as matrix material, to which gelatine is attached as a ligand.

3. Method for the preparation of column material for affinity chromatography containing gelatine as a ligand, characterized in that a watery solution of gelatine is mixed with a watery suspension of active carbon particles until the gelatine is adsorbed by the active carbon particles, after which superfluous gelatine is disposed of by washing it out.

4. Method according to conclusion 3, characterized in that the suspension of active carbon particles is degasified by heating before mixing with the gelatine solution.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CHEMICAL ABSTRACTS, vol. 107, no. 13, 28th September 1987, page 308, abstract no. 111979y, Columbus, Ohio, US; R.M. TOLON et al.: "Purification of fibronectin using gelatin derivatives", & REV. ESP. FISIOL. 1986, 42(4), 535-41 * Abstract * | 1 | C 07 K 3/20 B 01 D 15/08 C 07 K 15/14 |
| Y | CHEMICAL ABSTRACTS, vol. 87, no. 6, 8th August 1977, page 315, abstract no. 44200r, Columbus, Ohio, US; N. NAKABAYASHI et al.: "Encapsulation of activated charcoal beads with gelatins for direct hemoperfusion", & KOBUNSHI RONBUNSHU 1977, 34(4), 323-30 * Abstract * | 1 | |
| X | Idem | 2-4 | |
| Y | CHEMICAL ABSTRACTS, vol. 84, no. 19, 10th May 1976, page 340, abstract no. 140491v, Columbus, Ohio, US; & JP-A-75 144 684 (NIPPON STEEL CORP.) 20-11-1975 * Abstract * | 1 | TECHNICAL FIELDS SEARCHED (Int. Cl.5) |
| X | Idem | 2-4 | C 07 K A 61 K B 01 D |

The present search report has been drawn up for all claims

| Place of search | Date of completion of the search | Examiner |
|---|---|---|
| THE HAGUE | 08-11-1989 | RYCKEBOSCH A.O.A. |